# EUROPÄISCHE PATENTSCHRIFT

(11) **EP 0 922 450 B1**
(45) Veröffentlichungstag und Bekanntmachung des Hinweises auf die Patenterteilung: **23.04.2003**
(21) Anmeldenummer: 98121750.8
(22) Anmeldetag: 16.11.1998
(51) Int. Cl.: A61K 9/20, A61K 35/78

(54) **Brausezubereitung enthaltend ein Pflanzenextrakt**
Effervescent preparation containing a plant extract
Composition effervescente contenant un extrait de plantes

(30) Priorität: 01.12.1997 EP 97121045
(43) Veröffentlichungstag der Anmeldung: 16.06.1999
(73) Patentinhaber: Dr. Gergely & Co., 1053 Wien (AT)
(72) Erfinder: Gergely, Gerhard Dr., 1053 Wien (AT); Gergely, Thomas Dr., 1053 Wien (AT); Gergely, Irmgard, 1053 Wien (AT); Gergely, Stefan Dr., 1053 Wien (AT)
(74) Vertreter: Büchel, Kurt F.

(56) Entgegenhaltungen:
- EP-A- 0 242 794
- EP-A- 0 743 062
- WO-A-87/01936
- WO-A-90/03179
- WO-A-97/29642
- DE-A- 4 233 575
- DE-A- 19 509 856
- CHEMICAL ABSTRACTS, vol. 127, no. 3, 21. Juli 1997 Columbus, Ohio, US; abstract no. 39869, KOBAYASHI, NORIHIKO ET AL: "Effervescent tablets containing herbal medicines" XP002096826 & JP 09 077675 A (BAYER YAKUHIN, LTD., JAPAN;FUJI YAKUHIN K. K.)

## Beschreibung

Die Erfindung betrifft eine Brausezubereitung nach dem Oberbegriff des Anspruches 1. Fast alle Pflanzen-Trockenstoffteilchen, seien es nun Extrakte oder getrocknete Preßsäfte, beeinflussen die Auflösung, allerdings - in Abhängigkeit von den charakteristischen Inhaltsstoffen, meist Füllstoffen wie Zucker o.dgl. - mehr oder weniger stark. Einige Pflanzenextrakte erfordern aufgrund ihrer stark verzögernden Wirkung der Auflösung in Löstabletten oder Brausetabletten ganz besondere Maßnahmen, um diese Eigenschaften zu kompensieren. Das Problem der Verschleimung von Pflanzenextrakten, die deshalb schlechte Auflösungseigenschaften in wässriger Lösung zeigen, wird z.B. für eine solche Brausezubereitung in der EP-A1 97118648.1 dadurch umgangen, daß die Pflanzenextrakt-Teilchen mit einer dünnen Schicht einer öligen, fettigen oder wachsartigen Substanz beschichtet werden. Dies ist aber ein zusätzlicher Arbeitsschritt, und ist auch wegen des Einbringens solcher Substanzen in die zum Trinken bestimmte Brauselösung nicht in allen Fällen erwünscht.

Es gibt jedoch auch andere Trocken-Extrakte, wie Silymarin, Cimicifuga, gering dosierte Efeuextrakte und Echinacea-Extrakte, die zwar die Auflösung einer löslichen Darreichungsform, wie einer Lös- oder Brausetablette, geringfügig verlangsamen, jedoch nicht in einem solchen Ausmaß, daß die erwähnten, extremen Maßnahmen erforderlich sind.

Nun hat man auch schon vorgeschlagen, dem flüssigen Pflanzenextrakt vor seiner Trocknung bis zu 20% Mono- oder Disaccaride zuzusetzen (EP-A1 743 062), oder man hat sich mit der Mischung von Pflanzenextraktteilchen mit den Komponenten der Brausebasis (WO 97/2064) zufrieden gegeben. Beides ergibt keine oder nur eine beschränkte Lösung des eingangs geschilderten Problems:

Zucker ist einerseits aus Kaloriengründen in pharmazeutischen Zubereitungen eher unerwünscht; andererseits ist er leicht und schnell löslich, wodurch das Verschleimen noch begünstigt wird. Aber selbst langsamer lösliche Zucker, wie z.B. Lactose, können in den geringen dort vorgeschlagenen Mengen - insbesondere in der innigen Mischung mit den Trockenstoffteilchen (die ja aus der Lösung durch Vakuum- oder Sprühtrocknung gebildet werden) - nicht wirklich die Verschleimung und damit die extrem lange Auflösungszeit einer Brausetablette verhindern. Ebenso leicht und schnell sind die Komponenten der Brausebasis löslich.

Darüberhinaus haben beide genannten Brausezubereitungen der Zusammensetzung und Menge der Brausebasis kaum Bedeutung beigemessen. Diese ist aber für eine gut lösliche, Pflanzenextrakt enthaltende Brausetablette von größter Bedeutung.

Die WO 90/03179 offenbart eine Brausetablette, die einen hochkonzentrierten Extrakt Salicis enthält. Als Bindemittel wird Polyäthylenglykol 6000 und Polyvinylpyrrolidon eingesetzt. Das Gewichtsverhältnis des Extrakts zur Brausebasis beträgt 1:3,2 bis 1:3,44.

Die wesentlichsten Darreichungsformen für Phytopharmaka sind derzeit Tabletten, Dragees oder Tropfen, wie z. B. für Echinacea, Silymarin, Harpagophytum, Herzgespannkraut, Schöllkraut, Efeu u.a.

Die Erfindung hat sich nun die Aufgabe gestellt, eine lösliche Darreichungsform für Pflanzen-Trockenextrakte bzw. getrockneten Pflanzen-Preßsaft zu entwickeln, die alle erwähnten Nachteile vermeiden und sich in Wasser von 15 bis 18°C innerhalb von höchstens 3 min auflösen. Diese Aufgabe wird erstmals in überraschender Weise durch die Kombination der im Kennzeichen des Anspruches 1 genannten Merkmale gelöst. Details und Weiterbildungen des Erfindungsgedankens sind in den abhängigen Ansprüchen beschrieben.

Einerseits sollen die Pflanzenextrakt-Teilchen mit solchen pharmazeutisch zulässigen Hilfs- bzw. Füllstoffen versetzt werden, die zwar löslich sind, sich aber nicht so schnell und leicht wie Zucker lösen dürfen, da sie dann das Verschleimen der Pflanzenextrakt-Teilchen und damit eine Verlangsamung der Auflösung nicht oder zu wenig verhindern. Andererseits sollen sie auch nicht so schwer löslich wie Dextrin sein, weil im Falle der entsprechend erforderlichen Menge von Dextrin keine klare Lösung erzielt werden kann und andererseits das Aufziehen von fettigen, öligen oder wachsartigen Substanzen einen zusätzlichen Arbeitsschritt bedeutet und technologisch insofern aufwendig ist, als man mit organischen Lösungsmitteln arbeiten muß; außerdem ist dieser Schritt bei einer Reihe von Pflanzenextrakten nicht unbedingt erforderlich, wenn man eine Zusammensetzung entsprechend dem Erfindungsgedanken wählt.

Darüber hinaus ist es wie erwähnt erfindungswesentlich, daß die Trockenstoffteilchen *neben* dem wasserlöslichen, pharmazeutisch zulässigen Füllstoff - vorzugsweise neben 40 bis 250 Gewichtsteilen Füllstoff bezogen auf 100 Gewichtsteile Trockenstoff - vorliegen. Als solcher Füllstoff haben sich im Sinne der Erfindung Mannit und/oder Lactose als besonders zweckmäßig erwiesen. Mittels der Erfindung kann das Auflöseverhalten sogar handelsüblicher Trockenstoffteilchen, die häufig - wie erwähnt durch Vakuum- oder Sprühtrocknung - auf einem leicht löslichen Träger aufgezogen und so mit diesem innig gemischt sind, beträchtlich verbessert werden.

Eine größere Menge Füllstoff, dessen Teilchen neben den Trockenstoffteilchen, und damit zwischen diesen und den Komponenten des Brausesystems vorliegen, verhindert nämlich, daß die Reaktion der Brausekomponenten miteinander durch die beim Wasserzutritt sehr rasch eintretende Verschleimung der Oberfläche der Trockenstoffteilchen gebremst wird. Ansonsten ergeben sich nämlich Auflösezeiten einer solchen Brausetablette von über 5, teilweise bis zu 15 min und darüber, was natürlich für den praktischen Gebrauch nicht akzeptabel ist.

Andererseits hat sich gezeigt, daß man durchaus zufriedenstellende Auflösungseigenschaften und Auflösungszeiten dann erhält, wenn man die Extrakte mit einer Brausebasis aus C02-abspaltenden Substanzen und eßbaren, organischen Säuren, bzw. deren Salzen vermischt, wobei das Verhältnis von Säuren bzw. deren Salzen zu den C02-abspaltenden Substanzen 1:0,3 bzw. 1:0,9 beträgt, sodaß ein pH-Wert von 3,8 bis 5,6 resultiert. Als alkalische, CO2-abspaltende Bestandteile können sämtliche Alkalihydrogencarbonate und/oder - carbonate, wie Kaliumhydrogencarbonat, Kaliumcarbonat, Natriumhydrogencarbonat, Natriumcarbonat, Natriumglycincarbonat u.ä. eingesetzt werden, als Säuren insbesondere Zitronensäure, Weinsäure, Äpfelsäure und/oder deren sauren Salze. Sowohl die Alkali- als auch die sauren Komponenten können entweder einzeln oder auch in Mischung vorliegen.

Eine zweite wesentliche Komponente der Erfindung ist das Verhältnis des Extraktes zu der Gesamtmenge der Brausebasis, das - um eine Auflösungszeit von höchstens 3 min zu erreichen - ein Verhältnis von 1 Gewichtsteil Pflanzen-Trockenextrakt zu 3 bzw. 25 Gewichtsteilen der Brausekomponenten aufweist. Verhältnisse, bei denen der Anteil der Brausekomponenten in der fertigen Darreichungsform wesentlich über 25 Gewichtsteilen liegt, bringen interessanterweise keine signifikante Beschleunigung der Auflösung, sondern es resultiert nur ein - in der Regel unerwünschtes - größeres Tablettengewicht und damit auch eine weniger rationelle Herstellung, und zwar insbesondere aufgrund des höheren Rohstoffeinsatzes und der damit unwirtschaftlicheren und teureren Formulierung. Werden andererseits in der Rezeptur weniger als 3 Gewichtsteile Brausebasis auf 1 Gewichtsteil Extrakt eingesetzt, so macht sich dies bereits in einer signifikanten Verlangsamung der Auflösungszeit durch den Einfluß der Pflanzen-Trockenextrakte bemerkbar.

Z.B. zeigt eine Echinacea-Brause- bzw. Löstablette, bei der weniger als 3 Gewichtsteile Brausebasis auf 1 Gewichtsteil Extrakt vorliegen, bei einer Härte von 7,5 bereits eine Auflösungszeit von über 3 min, während eine erfindungsgemäß hergestellte Brausetablette (mit einem Verhältnis von Echinacea-Trockenextrakt zu Brausebasis 1:10) eine Auflösungszeit von nur 80 sec bis 1 ½ min zeigt. Beträgt das Verhältnis der Brausebasis zum Echinacea-Trockenextrakt jedoch mehr als 25 (bei gleichem Tablettengewicht und gleicher Härte), so ergibt sich dabei immer noch dieselbe Auflösungszeit von ca. 80 sec. Man sieht somit, daß selbst bei einer auf etwas mehr als das 2 1/2-fache vergrößerten Brausebasismenge keine wesentlich schnellere Auflösungszeit resultiert. In diesem Fall befindet sich aber bei gleichem Tablettengewicht nur mehr ein kleiner Teil der Extraktmenge in der Tablette, z.B. statt 250 mg nur mehr 105 mg. Somit müßten 2 ½ Tabletten verabreicht werden, um die entsprechende Dosis zu erzielen. Bei gleichbleibender Dosis und bei einem Verhältnis von 1 Teil Trockenextrakt zu über 25 Teilen Brausebasis würde bei einer Dosierung von 250 mg Trockenextrakt pro Dosis der Brausebasisanteil bereits über 6 g betragen und wäre somit nicht mehr wirtschaftlich und teuer.

In einem pH-Bereich über 5,6 erfolgt die Reaktion zwischen den Säurebestandteilen und den Alkalibestandteilen des Brausesystems bereits langsamer, sodaß durch den zusätzlichen Einfluß der Pflanzenextrakte die Auflösungszeit weiter verlängert wird. Bei einem saureren pH-Wertals 3,8 ist wieder nicht genügend C02-abspaltender Alkalibestandteil vorhanden, um den verzögernden Einfluß der Trockenextrakte auf die Auflösungszeit durch eine vehemente Reaktion der beiden Brause-Bestandteile zu kompensieren.

Bei einer Echinacea-Brausetablette, mit einem Verhältnis von Echinacea-Trockenextrakt zu Brausebasis von 1:6,5, die ein pH-Wert von 3,6 zeigt, beträgt die Auflösungszeit bereits mehr als 2 min 50 sec, meist etwa 3 min. Aber auch bei einem gleichen Verhältnis von Trockenextrakt zu Brausebasis, jedoch mit einem höheren Anteil an alkalischen C02-abspaltenden Bestandteilen, wobei ein pH-Wert von 5,65 resultiert, beträgt die Auflösungszeit bei einer Härte von 7,5 bereits 2 min und darüber.

Es kann erforderlich sein, C02-abspaltende Alkalibestandteile sowie auch saure Bestandteile in Mischung zu bringen, um einen pH-Wert von 4 bis ca.5,5 zu erreichen; z.B. würde bei Mononatriumcitrat und Natriumhydrogencarbonat ein Verhältnis von 1:0,15 ausreichen, um einen pH-Wert von 4 zu erzielen. Dies würde jedoch nicht ausreichen, um eine entsprechende Auflösungsgeschwindigkeit der Tablette zu erzielen. Gleiches gilt z.B. für Mononatriumtartrat, wo auf 1 Gewichtsteil nur 0,2 Gewichtsteile Natriumbicarbonat erforderlich sind, um einen pH-Wert von 4 zu erreichen, womit aber wiederum nicht die entsprechenden Auflösungseigenschaften erzielt werden können.

Als Extrakte können alle gängigen Pflanzen-Trockenextrakte eingesetzt werden, wie z.B. native, d.h. 100%ige Extrakte, oder aber auch 70%ig-, 60%ig- oder 50%ig sprühgetocknete Extrakte mit z.B. Maltodextrin, Calciumphosphat u.a., bevorzugt aber nicht mit Zucker. Die Mengenangabe der Verhältnisse der Extrakte zur Brausebasis bezieht sich auf den jeweils eingesetzten Extrakt, unabhängig, ob er nativ, d.h. 100%ig ist oder eine andere Konzentration aufweist. Es können auch mehrere Extrakte in Mischung in die erfindungsgemäße Brausezubereitung eingebracht werden, ebenso wie auch die Extrakte mit anderen Wirkstoffen kombiniert werden können. Als Beispiel kann hier eine Echinacea-Brausetablette in einer Dosierung von 250 bis 600 mg Trockenextrakt pro Tablette in Kombination mit Zink angeführt werden.

Die Anwendung der Echinacea erfolgt zur unterstützenden Therapie bei grippalen Infekten und beruht auf den ihr zugesagten immunstimulierenden Eigenschaften. Es zeigte sich bei klinischen Studien eine schnellere Besserung der Symptome eines grippalen Infektes des oberen Respirationstraktes, und die Krankheitsdauer verringerte sich. Grundsätzlich hat zwar auch schon Zink bei diesem Indikationsgebiet Anwendung gefunden, doch konnte im vorliegenden Fall eine unerwartete, synergistische Wirkung festgestellt werden. Zink kann in Form der anorganischen oder organischen Salze eingesetzt werden, wie z.B. als Zinksulfat, -orotat, -glycerophosphat oder -acetat in einer Menge von 5 - 50mg Zinkion pro Dosis.

In die erfindungsgemäße Brausezubereitung können zur Geschmacksverbesserung künstliche Süßstoffe, wie Natriumcyclamat, Saccharin-Natrium, Aspartam, Acesulfam und Neohesperidin, eingebracht werden, vorzugsweise aber - wie oben erwähnt - nicht Zucker, Fructose od.dgl. Hingegen ist der Zusatz von pharmazeutisch oder lebensmittelrechtlich zugelassenen Hilfsstoffen, die die oben erwähnten Bedingungen erfüllen, durchaus erwünscht, wie z.B. von Zukkeralkoholen (Mannit ist gegenüber Sorbit bevorzugtl), weniger schnell bzw. leicht löslichen Kohlehydraten, wie z.B. Lactose, oder aber auch von Maltodextrin, etc.

Nach Bedarf können auch - um die Auflösung der Pflanzen-Trockenextrakte zu verbessern - Tenside zugefügt werden. Um die Schaumbildung beim Auflösen zu verhindern und die Auflösung der Tablette zu beschleunigen, können vorteilhafter Weise Anti-Schaummittel in die Formulierung eingebaut werden. Die Tenside oder Anti-Schaummittel können auf den Pflanzen-Trockenextrakt direkt aufgebracht oder in Form einer getrennten Phase der Mischung - z.B. vor dem Verpressen zu Tabletten - zugesetzt werden. Zu diesem Zweck werden Tenside und/oder Anti-Schaummittel auf einen der erfindungsgemäß vorgesehenen, neutralen Hilfs- bzw. Füllstoffe aufgezogen und der Tabletten-Endmischung zugefügt. Zur Herstellung der Anti-Schaummittel-Phase wird das Anti-Schaummittel mittels eines Lösungsmittels oder einer wässrigen Suspension auf einen Füllstoff aufgezogen. Anschließend wird das Lösungsmittel verdampft, und man fügt diese Phase der Tabletten-Endmischung zu. Die eingebrachte Menge des Anti-Schaummittels hängt weitgehend vom Schaumverhalten des eingesetzten Pflanzenextraktes ab.

Die erfindungsgemäß hergestellte Pflanzenextrakt-Brausezubereitung zeichnet sich durch eine schnelle Auflösung in Wasser (Auflösungszeit bei 17°C unter 3 min) und durch ein geringes Schaumverhalten aus. Die Erfindung wird im folgenden an Hand einiger Beispiele näher erläutert:

### Beispiel 1 (Silymarin-Trockenextrakt)

Verhältnis Wirkstoff zu Brausebestandteilen 1:4.8, Säure zu Alkali 1:0.8. Herstellung: 1147 Gewichtsteile Zitronensäure werden auf 60°C erwärmt und mit 24 Gewichtsteilen einer 50%igen wässrigen Trinatriumcitrat-Lösung befeuchtet. Es erfolgt die Zugabe von 917 Gewichtsteilen Natriumhydrogencarbonat, die man unter Rühren anreagieren läßt. Anschließend wird die Mischung mit 16 Gewichtsteilen 50%iger Natriumcitrat-Lösung nochmals granuliert; sodann werden 70 Gewichtsteile Natriumcarbonat zugegeben. Das Granulat wird mittels Vakuum bei einer Temperatur von 50°C getrocknet und auf 2 mm gesiebt. Zu diesem gesiebten Brausegranulat werden 4 Gewichtsteile Aspartam, 20 Gewichtsteile Acesulfam sowie 460 Gewichtsteile Silymarin-Trockenextrakt, weiters 50,1 Gewichtsteile einer Mannit-Simethicon-Phase (0,1 Gewichtsteile Simethicon auf 50 Gewichtsteile Mannit), 40 Gewichtsteile Aroma sowie eine Mischung aus 0,8 Gewichtsteilen Natriumdioctylsulfosuccinat in 15 Gewichtsteilen Mannitol zugefügt.

Das Granulat wird zu Tabletten von 2,85 g verpreßt, die sich in Wasser innerhalb von 80 bis 90 sec auflösen. Die wässrige Lösung zeigt einen pH-Wert von 5,0-5,1.

### Beispiel 2 (Cimicifuga-Trockenextrakt)

Verhältnis Wirkstoff zu Brausebestandteilen: 1:20, Säure zu Alkali: 1:0.69. Herstellung: 482 Gewichtsteile Zitronensäure werden in einem Vakuumgranulator, dessen Manteltemperatur 90°C beträgt, unter Rühren auf 60°C erwärmt und mit einer Lösung aus 6 Gewichtsteilen Natriumcitrat in 6 Gewichtsteilen Wasser befeuchtet. Sodann werden 304 Gewichtsteile Natriumhydrogencarbonat zugegeben und kontrolliert anreagieren lassen; anschließend werden 28 Gewichtsteile Natriumcarbonat zugemischt. Das resultierende Granulat wird mittels Vakuum bei 50°C getrocknet und auf 2 mm gesiebt. Zu 820 Gewichtsteilen des Brausegranulates werden 41 Gewichtsteile Cimicifuga-Trockenextrakt sowie 100 Gewichtsteile Mannit, 40 Gewichtsteile Aroma und 3 Gewichtsteile Saccharin-Natrium, sowie 30 Gewichtsteile Natriumcyclamat zugefügt.

Das homogen vermischte Granulat wird zu Tabletten mit einem Gewicht von 1,03g verpreßt, die sich in Wasser innerhalb von 50 sec auflösen. Die wässrige Lösung zeigt einen pH-Wert von 4.8.

### Beispiel 3 (Echinacea + Zink)

Verhältnis Wirkstoff zu Brauseanteil 1:5.5, Säure zu Alkali 1:0,69. Herstellung: 1596 Gewichtsteile Zitronensäure und 41,5 Gewichtsteile Zinksulfat werden in einem beheizbaren Vakuumgranulator unter Rühren auf 60°C erwärmt. Diese Mischung wird mit 8 Gewichtsteilen einer Lösung befeuchtet, die aus 4 Gewichtsteilen Natriumcitrat.2H20 und 4 Gewichtsteilen Wasser besteht. Sodann werden 1005 Gewichtsteile Natriumhydrogencarbonat, 5 Gewichtsteile Aspartam und 9 Gewichtsteile Acesulfam zugegeben und kontrolliert anreagieren gelassen, und schließlich 92 Gewichtsteile Natriumcarbonat zugegeben.

Das Granulat wird abschließend mittels Vakuum bei 50°C getrocknet und auf 2 mm gesiebt. Dem Granulat werden 500 Gewichtsteile Echinacea-Trockenextrakt 70%ig sowie 110 Gewichtsteile Mannit, weiters 100 Gewichtsteile einer 1 %igen Mannit-Simethicon-Phase (auf 99 Gewichtsteile Mannit 1 Gewichtsteil Polysiloxan) sowie 60 Gewichtsteile Aroma zugesetzt, und alles wird homogen vermischt. Die fertige Mischung wird zu Tabletten von je 3,62 g verpreßt, die sich in Wasser innerhalb von 80 bis 90 sec auflösen. Die wässrige Lösung zeigt einen pH-Wert von 4,55 bis 4,70.

### Beispiel 4 (Echinacea-Brausetablette)

Verhältnis Wirkstoff zu Brauseanteil: 1:10, Säure zu Alkali: 1:0,69. Herstellung: In einen beheizbaren Vakuumgranulator werden 1400 Gewichtsteile Zitronensäure eingebracht, auf 50°C erwärmt und mit 5,5 Gewichtsteilen einer 50%igen Natriumcitrat-Lösung in Wasser befeuchtet. Anschließend werden 500 Gewichtsteile Natriumhydrogencarbonat und 380 Gewichtsteile Kaliumhydrogencarbonat sowie 1 Gewichtsteil Aspartam und 9 Gewichtsteile Acesulfam zugefügt, kontrolliert anreagieren lassen und anschließend 80 Gewichtsteile Natriumcarbonat zugegeben. Das feuchte Granulat wird bei 50°C getrocknet und anschließend auf 2 mm gesiebt. 2360 Gewichtsteile dieser Brausebasis werden mit 235 Gewichtsteilen eines 82%igen, getrockneten Echinacea-Preßsaftes versetzt; weiters werden 70 Gewichtsteile Lactose, 83 Gewichtsteile Mannit sowie 60 Gewichtsteile Aroma zugefügt.

Das Granulat wird zu Tabletten mit einem Tablettengewicht von 2,82 g verpreßt; die sich bei einer Härte von 7 bis 8 in Wasser innerhalb von 80 bis maximal 90 sec auflösen. Die wässrige Lösung zeigt einen pH-Wert von 4,65 bis 4,75.

### Beispiel 5 (Agni Casti-Brausetablette)

Verhältnis Wirkstoff zu Brauseanteil: 1:22, Säure zu Alkali: 1: 0.5.

Die Herstellung erfolgt analog dem vorgenannten Beispiel, wobei 586 Gewichtsteile Zitronensäure, 278 Gewichtsteile Natriumhydrogencarbonat sowie 16 Gewichtsteile Natriumcarbonat für die Brausebasis zum Einsatz kommen. 880 Gewichtsteile dieser Brausebasis werden mit 40 Gewichtsteilen des Agni Casti-Trockenextraktes sowie 100 Gewichtsteilen Mannit, 40 Gewichtsteilen Aroma und 3 Gewichtsteilen Saccharin sowie 30 Gewichtsteilen Natriumcyclamat vermischt.

Das Granulat wird zu Tabletten mit einem Tablettengewicht von je 1,05 g verpreßt, die sich in Wasser innerhalb von 60 bis 70 sec auflösen. Die wässrige Lösung zeigt einen pH-Wert von 4,0 - 4,1.

### Beispiel 6 (Echinacea-Brausetablette mit Weinsäure)

Verhältnis Wirkstoff zu Brauseanteil: 1:15, Säure zu Alkali: 1:0.88.

Herstellung: 1995 Gewichtsteile Weinsäure werden in einem beheizbaren Vakuumgranulator auf 55°C erwärmt und mit 10 Gewichtsteilen Wasser befeuchtet. Sodann werden 1482 Gewichtsteile Natriumhydrogencarbonat zugefügt, kontrolliert anreagieren gelassen und anschließend 273 Gewichtsteile Natriumcarbonat zugegeben. Das Granulat wird mittels Vakuum bei 50°C getrocknet und auf 2 mm gesiebt. Zu dem gesiebten Granulat werden 250 Gewichtsteile Echinacea-Trockenextrakt 70%ig sowie 100 Gewichtsteile Lactose, 9 Gewichtsteile Acesulfam, 5 Gewichtsteile Aspartam, 200 Gewichtsteile einer Mannit-Simethicon-Phase (2 Gewichtsteile Simethicon auf 98 Gewichtsteile Mannit), sowie schließlich 60 Gewichtsteile Aroma zugefügt und homogen vermischt.

Das Granulat wird zu Tabletten mit 4,37 g verpreßt, die sich in Wasser innerhalb von 110 sec auflösen. Die wässrige Lösung zeigt einen pH-Wert von 4,8.

### Beispiel 7 (Echinacea Brausetablette mit Zitronensäure /Mononatriumcitrat)

Verhältnis Wirkstoff zu Brauseanteil: 1:15, Säure zu Alkali: 1:0.42. Die Herstellung erfolgt analog dem vorangegangenen Beispiel, wobei für die Brausebasis als Säurekomponenten 1949 Gewichtsteile Zitronensäure und 687 Gewichtsteile Mononatriumcitrat, als Alkalikomponenten 928 Gewichtsteile Natriumhydrogencarbonat und 186 Gewichtsteile Natriumcarbonat eingesetzt werden.

Zu dem gesiebten Brausegranulat von 3750 Gewichtsteilen werden 250 Gewichtsteile Echinacea-Trockenextrakt 70%ig sowie 9 Gewichtsteile Acesulfam, 5 Gewichtsteile Aspartam, 100 Gewichtsteile Mannit-Simethicon-Phase (1 Gewichtsteil Simethicon auf 99 Gewichtsteile Mannit) sowie 60 Gewichtsteile Aroma zugefügt und homogen vermischt.

Das Granulat wird zu Tabletten von 4,17 g verpreßt, die sich in Wasser innerhalb von 70 sec auflösen. Die wässrige Lösung zeigt einen pH-Wert von 4.2.

### Beispiel 8 (Harpagophytum)

Verhältnis Wirkstoff zu Brausebasis: 1:2,58, Säure zu Alkali: 1:0,62 Es wird eine Brausebasis entsprechend dem Beispiel 5 hergestellt. 1550 Gew.teile dieser Brausebasis werden mit 600 Gew.teilen Harpagophytum-Extrakt 80%ig sowie 300 Gew.teilen einer 1 %igen Mannit-Simethicon-Phase, weiters mit 50 Gew.teilen einer 2%igen Mannit/Docusat-Phase sowie 20 Gew.teilen Lactose, 50 Gew.teilen Natriumsulfat, weiters 70 Gew.teilen Aspartam und 80 Gew.teilen pulverigem Aroma vermischt. Das Granulat wird zu Tabletten von je 2,72 g verpreßt, die sich bei einer Härte von 7 - 8 kp in Wasser innerhalb von 120 - 130 Sekunden auflösen und einen pH-Wert von 4,4 zeigen.

### Beispiel 9 (Herzgespannkraut-Extrakt)

Verhältnis Wirkstoff zu Brausebasis: 1:6,44, Säure zu Alkali: 1:0,8. Es wird eine Brausebasis entsprechend Beispiel 1 hergestellt; 1482 Gew.teile Brausebasis werden mit 20 Gew.teilen Herzgespannkraut-Extrakt, 5 Gew.teilen Docusat-Natrium, 100 Gew.teilen 0,4%iger Mannit/Simethicon-Phase sowie 30 Gew.teilen Zitronenaroma und schließlich 50 Gew.teilen Natriumcyclamat vermischt und zu Tabletten von je 1,9g verpreßt. Das Produkt zeigt nur eine geringe Schaumbildung und bei einer Härte von 9 kp eine Auflöszeit von 90 Sekunden. Die wässrige Lösung ergibt einen pH-Wert von 5,1.

### Beispiel 10 (Schöllkraut)

Verhältnis Wirkstoff zu Brausebasis: 1:5,85, Säure zu Alkali: 1:0,8. Es wird eine Brausebasis hergestellt entsprechend Beispiel 1. 1462 Gew.teile dieser Brausebasis werden mit 250 Gew.teilen Schöllkraut Extrakt, 5 Gew.teilen Docusat-Natrium sowie 100 Gew.teilen 0,4%iger Mannit/Simethicon-Phase und weiters 50 Gew.teilen Natriumcyclamat, 3 Gew.teilen Saccharin-Natrium und 30 Gew.teilen Zitronenaroma vermischt. Die Mischung wird zu Tabletten von je 1,9g verpreßt. Bei einer Härte von 6 kp lösen sich die Tabletten in 60 - 70 Sekunden mit nur einer geringen anfänglichen Schaumbildung auf und ergeben einen pH von 5,1.

## Patentansprüche

1. Brausezubereitung in Form von Granulat oder einer Tablette, enthaltend wenigstens einen wasserlöslichen oder zumindest suspendierbaren, pulver- oder granulatförmigen Trockenstoff in Form getrockneten Preßsafts und/oder, vorzugsweise, getrockneten Extrakts bestimmter Pflanzenteile, und eine Brausebasis aus wenigstens einer festen, eßbaren, organischen Säure und/oder deren saurem Salz, sowie wenigstens einem Alkali- und/oder Erdalkalicarbonat bzw. -hydrogencarbonat, **dadurch gekennzeichnet, daß**
a) die Trockenstoffteilchen neben einem wasserlöslichen, pharmazeutisch zulässigen Füllstoffvorliegen;
b) das Gewichtsverhältnis der Trockenstoffe zur Brausebasis zwischen 1:5 und 1:25 liegt; und
c) das Gewichtsverhältnis der sauren zu den CO₂-abspaltenden Komponenten der Brausebasis zwischen 1:0.3 und 1:0.9 liegt, so dass sich mit dem Pflanzenextrakt in wässriger Lösung oder Suspension ein pH-Wert von 3.8 bis 5.6 ergibt.

2. Brausezubereitung nach Anspruch 1, **dadurch gekennzeichnet, dass** 40 bis 250 Gewichtsteile Füllstoff bezogen auf 100 Gewichtsteile Trockenstoff vorliegen

3. Brausezubereitung nach Anspruch 1 oder 2, **dadurch gekennzeichnet, daß** der Füllstoff in Wasser leichter löslich ist als Dextrin.

4. Brausezubereitung nach Anspruch 3, **dadurch gekennzeichnet, dass** der Füllstoff Maltodextrin, Mannit und/oder Lactose ist.

5. Brausezubereitung nach einem der vorhergehenden Ansprüche, **dadurch gekennzeichnet, daß** das Gewichtsverhältnis der Trockenstoffe zur Brausebasis zwischen 1:5 und 1: 20 liegt.

6. Brausezubereitung nach einem der vorhergehenden Ansprüche, **dadurch gekennzeichnet, daß** die Trockenstoffe von Silymarin, Cimicifuga, Echinacea, Efeu, Harpagophytum, Herzgespannkraut, Schöllkraut oder Agni Casti stammen.

7. Brausezubereitung nach einem der vorhergehenden Ansprüche, **dadurch gekennzeichnet, daß** sie außerdem Spurenelemente, insbesondere im Falle des Pflanzenextraktes Echinacea eine wasserlösliche Zinkverbindung, bevorzugt etwa 5 bis etwa 50 mg Zinkion pro Tablette, enthält.

8. Brausezubereitung nach einem der vorhergehenden Ansprüche, **dadurch gekennzeichnet, daß** sie außerdem ein Anti-Schaummittel enthält, insbesondere Dimethicon oder Simethicon, bevorzugt in einer Menge von 0,05 - 5, insbesondere 0,1 - 2.0 Gewichtsteile auf 100 Gewichtsteile des Pflanzenextraktes bezogen.

9. Verfahren zur Herstellung einer Brausezubereitung nach einem der vorhergehenden Ansprüche, **dadurch gekennzeichnet, daß** wasserlöslicher oder zumindest suspendierbarer, pulver- oder granulatförmiger Trockenstoff in Form getrockneten Preßsafts und/oder getrockneten Extrakts bestimmter Pflanzenteile mit einem wasserlöslichen, pharmazeutisch zulässigen Füllstoffes, vorzugsweise 40 bis 250 Gewichtsteilen Füllstoff bezogen auf 100 Gewichtsteile Trockenstoff, und Brausekomponenten, nämlich wenigstens einer festen, eßbaren, organischen Säure und/oder deren saurem Salz, sowie wenigstens einem Alkali- und/oder Erdalkalicarbonat bzw. -hydrogencarbonat gemischt werden.

## Claims

1. Effervescent preparation in the form of granules or of a tablet, containing at least one water-soluble or at least suspendable, pulverulent or granular dry substance in the form of a dried pressed juice and/or, preferably, dried extract of certain plant parts, and an effervescent base comprising at least one solid, edible, organic acid and/or an acidic salt thereof, and at least one alkali metal carbonate and/or alkaline earth metal carbonate and/or alkali metal bicarbonate and/or alkaline earth metal bicarbonate, **characterized in that**
a) the particles of dry substance are present in addition to a water-soluble, pharmaceutically permissible filler;
b) the weight ratio of the dry substances to the effervescent base is from 1:5 to 1:25; and
c) the weight ratio of the acidic components to the CO₂-eliminating components of the effervescent base is from 1:0.3 to 1:0.9, so that the plant extract in aqueous solution or suspension results in a pH of from 3.8 to 5.6.

2. Effervescent preparation according to Claim 1, **characterized in that** from 40 to 250 parts by weight of filler, based on 100 parts by weight of dry substance, are present.

3. Effervescent preparation according to Claim 1 or 2, **characterized in that** the filler is more freely soluble than dextrin in water.

4. Effervescent preparation according to Claim 3, **characterized in that** the filler is maltodextrin, mannitol and/or lactose.

5. Effervescent preparation according to any of the preceding Claims, **characterized in that** the weight ratio of the dry substances to the effervescent base is from 1:5 to 1:20.

6. Effervescent preparation according to any of the preceding Claims, **characterized in that** the dry substances originate from silymarin, cimicifuga, echinacea, ivy, harpagophytum, motherwort, common celandine or agni casti.

7. Effervescent preparation according to any of the preceding Claims, **characterized in that** it also contains trace elements, in particular in the case of the plant extract echinacea, a water-soluble zinc compound, preferably about 5 to about 50 mg of zinc ions per table.

8. Effervescent preparation according to any of the preceding Claims, **characterized in that** it also contains an antifoam, in particular dimethicone or simethicone, preferably in an amount of 0.05 - 5, in particular 0.1 - 2.0, parts by weight, based on 100 parts by weight of the plant extract.

9. Process for the production of an effervescent preparation according to any of the preceding Claims, **characterized in that** water-soluble or at least suspendable, pulverulent or granular dry substance in the form of a dried pressed juice and/or dried extract of certain plant parts is mixed with a water-soluble, pharmaceutically permissible filler, preferably from 40 to 250 parts by weight of filler, based on 100 parts by weight of dry substance, and effervescent components, namely at least one solid, edible, organic acid and/or an acidic salt thereof, and at least one alkali metal carbonate and/or alkaline earth metal carbonate and/or alkali metal bicarbonate and/or alkaline earth metal bicarbonate.

## Revendications

1. Préparation effervescente sous la forme de granulés ou d'un comprimé, contenant au moins une matière sèche qui est sous forme de poudre ou de granulés, qui est hydrosoluble ou du moins susceptible d'être mise en suspension dans l'eau et qui provient du séchage d'un jus de pressage et / ou, de préférence, d'un extrait de certaines parties de plantes et une base effervescente constituée par au moins un acide organique solide de qualité alimentaire et / ou son sel acide, ainsi que par au moins un carbonate ou un hydrogénocarbonate d'un métal alcalin ou alcalino-terreux, **caractérisée en ce que**
a) les particules de matière sèche sont présentes à côté d'un excipient hydrosoluble acceptable sur le plan pharmaceutique ;
b) le rapport pondéral de la matière sèche sur la base effervescente est entre 1 : 5 et 1 : 25 ; et
c) le rapport pondéral de l'acide sur le composant capable de libérer du CO₂ de la base effervescente se situe entre 1 : 0,3 et 1 : 0,9, de manière à ce que l'extrait de plantes en solution ou en suspension dans l'eau donne une valeur de pH de 3,8 à 5,6.

2. Préparation effervescente selon la revendication 1, **caractérisée en ce que** de 40 à 250 parties en poids d'excipient sont présentes pour 100 parties en poids de matière sèche.

3. Préparation effervescente selon la revendication 1 ou la revendication 2, **caractérisée en ce que** l'excipient est plus facilement dissout dans l'eau que la dextrine.

4. Préparation effervescente selon la revendication 3, **caractérisée en ce que** l'excipient est constitué par la maltodextrine, le mannitol et / ou le lactose.

5. Préparation effervescente selon l'une des revendications précédentes, **caractérisé en ce que** le rapport pondéral de la matière sèche sur la base effervescente se situe entre 1 : 5 et 1 : 20.

6. Préparation effervescente selon l'une des revendications précédentes **caractérisée en ce que** la matière sèche provient de la silymarine, de la cimicaire à grappes, de l'échinacée, du lierre, de l'harpagophyte étalé, de l'aviculaire, de la chélidoine ou de l'agni casti.

7. Préparation effervescente selon l'une des revendications précédentes, **caractérisée en ce qu'**elle contient, en outre, des oligo-éléments, en particulier dans le cas de l'extrait végétal de l'échinacée un composés soluble du zinc, de préférence d'environ 5 à environ 50 mg d'ions zinc par comprimé.

8. Préparation effervescente selon l'une des revendications précédentes, **caractérisée en ce qu'**elle contient, en outre, un agent antimousse, en particulier du diméthicone ou du siméthicone, de préférence en une quantité de 0,05 - 5 et, en particulier de 0,1 - 2,0 parties en poids pour 100 parties en poids de l'extrait de plante.

9. Procédé pour préparer une composition effervescente selon l'une des revendications précédentes, **caractérisé en ce que** l'on mélange une matière sèche qui est sous forme de poudre ou de granulés, qui est hydrosoluble ou du moins susceptible d'être mise en suspension dans l'eau et qui provient du séchage d'un jus de pressage et / ou d'un extrait de certaines parties de plantes avec un excipient hydrosoluble acceptable sur le plan pharmaceutique, de préférence à raison de 40 à 250 parties en poids d'excipient pour 100 parties en poids de matière sèche et des composants effervescents, en l'occurrence au moins un acide organique solide de qualité alimentaire et / ou son sel acide, ainsi qu'au moins un carbonate ou un hydrogénocarbonate d'un métal alcalin ou alcalino-terreux.
